(19) Europäisches Patentamt
European Patent Office
Office européen des brevets

(11) Publication number: **0 269 991 A1**

(12) **EUROPEAN PATENT APPLICATION**

(21) Application number: 87117397.7

(22) Date of filing: 25.11.87

(51) Int. Cl.[4]: C07D 453/02

(30) Priority: 28.11.86 JP 281891/86

(43) Date of publication of application: 08.06.88 Bulletin 88/23

(84) Designated Contracting States: AT BE CH DE ES FR GB GR IT LI LU NL SE

(71) Applicant: Eisai Chemical Co., Ltd.
22, Sunayama Hazaki-cho
Kashima-gun Ibaraki Prefecture(JP)

(72) Inventor: Minami, Norio
2-2, Kikusui-cho
Kawachinagano-shi Osaka(JP)
Inventor: Konishi, Masayuki
10000-76, Yatabe Hasaki-machi
Kashima-gun Ibaraki Pref.(JP)
Inventor: Kanai, Takeo
4680-103, Kyuchu Kashima-machi
Kashima-gun Ibaraki Pref.(JP)

(74) Representative: Hansen, Bernd, Dr.rer.nat. et al
Hoffmann, Eitle & Partner Patentanwälte
Arabellastrasse 4
D-8000 München 81(DE)

(54) **Process for the synthesis of 4-cyanoquinuclidine or a salt thereof.**

(57) 4-Cyanoquinuclidine or a salt thereof is synthetically prepared by reacting a compound represented by the following formula (I):

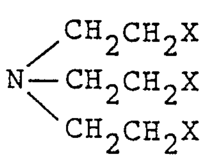

(I)

wherein X means a halogen atom, or a salt thereof with acetonitrile in the presence of a base.

EP 0 269 991 A1

# PROCESS FOR THE SYNTHESIS OF 4-CYANOQUINUCLIDINE OR A SALT THEREOF

## BACKGROUND OF THE INVENTION

1) Field of the Invention:

This invention relates to a novel process for the synthesis of 4-cyanoquinuclidine or a salt thereof.

2) Description of the Related Art:

4-Cyanoquinuclidine is useful as a synthesis intermediate for pharmaceutical products, chemical reagents and the like. Its synthesis is however not easy due to its unique structure. Its synthesis has been reported, for example, in "Helvetica Chimica Acta", 194, 1672-1679 (1954) and ibid., 195, 1680-1688 (1954). In these publications, 4-cyanoquinuclidine is synthesized via N-methyl-4-carbamoylpiperidine, N-methyl-4-cyanopiperidine and N-methyl-4-cyanoquinuclidine when 4-carbamoylpiperidine is used as a starting material. This process is however accompanied by drawbacks in that many steps are required and the yield of the step for converting the piperidine into the quinuclidine is as low as 17%.

## SUMMARY OF THE INVENTION

The present inventors have carried out an extensive investigation with a view toward developing a process for the synthesis of 4-cyanoquinuclidine. As a result, it has been found that the above compound can be synthesized by a one-step reaction of a tri(halogenoethyl)amine and acetonitrile, leading to completion of this invention.

An object of this invention is therefore to provide an industrially-excellent process for the synthesis of 4-cyanoquinuclidine or a salt thereof.

In one aspect of this invention, there is thus provided a process for the synthesis of 4-cyanoquinuclidine or a salt thereof, which comprises reacting a compound represented by the following formula (I):

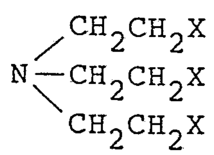

(I)

wherein X means a halogen atom, or a salt thereof with acetonitrile in the presence of a base.

The process of this invention can synthetically prepare 4-cyanoquinuclidine or a salt thereof by a one-step reaction of a tri(halogenoethyl)amine or a salt thereof and acetonitrile only, and is excellent from the industrial standpoint.

The above and other objects, features and advantages of the present invention will become apparent from the following description and the appended claims.

## DETAILED DESCRIPTION OF THE INVENTION AND PREFERRED EMBODIMENT

As the halogen atom represented by X in the formula (I), a chlorine atom, bromine atom, iodine atom or the like may be mentioned. Illustrative examples of the salt of the compound represented by the formula (I) may include the inorganic acid salts such as hydrochloride, hydrobromide, hydroiodide, sulfate, carbonate and bicarbonate; the organic carboxylates such as acetate, maleate, lactate, tartrate and trifluoroacetate; the organic sulfonates such as methanesulfonate, benzenesulfonate and toluenesulfonate; etc.

As exemplary bases useful in the practice of this invention, may be mentioned alkali metal amides such as sodium amide, potassium amide and lithium amide; and alkali metal hydrides such as sodium hydride, potassium hydride and lithium hydride.

The process of this invention can be carried out in an inert solvent at 0°C - the reflux temperature of the solvent. As the inert solvent, may be mentioned that not impairing the reaction, for example, an ether such as tetrahydrofuran, dioxane, ethyl ether, 1,2-dimethoxyethane or isopropyl ether; a hydrocarbon such as benzene, toluene, xylene or cyclohexane; dimethylsulfoxide; dimethylformamide; or a mixture thereof.

As exemplary salts of 4-cyanoquinuclidine, may be mentioned those referred to above as illustrative examples of the salt of the compound represented by the formula (I).

The present invention will hereinafter be described in further detail by the following Examples.

Example 1:

<u>4-Cyanoquinuclidine</u>

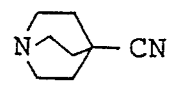

Trichloroethylamine (618 g) was dissolved in tetrahydrofuran (6,180 mℓ), followed by an addition of sodium amide (468 g). To the resulting solution, a mixed solution of acetonitrile (160 g) and tetrahydrofuran (1,600 mℓ) was added dropwise under stirring over 4 hours, and the reaction mixture was stirred for further one hour. The solvent was distilled off. Water (1,200 mℓ) was added to the residue and the resulting aqueous mixture was extracted twice with 600-mℓ portions of chloroform. The extract was concentrated to dryness to obtain 268 g of 4-cyanoquinuclidine in a crude form (yield of the crude product: 65.7%).

The crude 4-cyanoquinuclidine was sublimated and purified at 100 - 150°C and 20 mmHg, thereby obtaining 130 g of 4-cyanoquinuclidine in a purified form (yield: 31.8%).

Melting point: 104 - 106°C.
IR spectrum ($cm^{-1}$ KBr): 2225
NMR spectrum ( δ , $CDCl_3$ ):
1.5 - 2.1 (6H, m, ($A_2B_2$)),
2.6 - 3.2 (6H, m, ($A_2B_2$)).

Example 2:

<u>4-Cyanoquinuclidine p-toluenesulfonate</u>

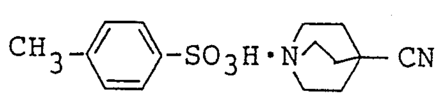

Sodium amide (176 g) was suspended in 1,2-dimethoxyethane (2,400 mℓ), followed by an addition of trichloroethylamine hydrochloride (241 g). To the resulting solution, a mixed solution of acetonitrile (45 g) and 1,2-dimethoxyethane (450 mℓ) was added dropwise under stirring over 4 hours, and the reaction mixture was stirred for further one hour. The reaction mixture was poured into chilled water (540 mℓ) and the organic layer was collected. Anhydrous magnesium sulfate (150 g) was added to the organic layer to dry same. The magnesium sulfate was filtered off and the filtrate was added with a mixture of p-toluenesulfonic acid hydrate (190 g) and 1,2-dimethoxyethane (950 mℓ). The resulting crystals were collected by filtration, washed with isopropyl ether, and then dried to obtain 321 g of the desired product (yield: 85.2%).

Melting point: 243 - 247°C.
IR spectrum ($cm^{-1}$,KBr): 2220

NMR spectrum ( δ, DMSO-$d_6$ ):
2.0 - 2.3 (6H, m),
2.27 (3H, s),
3.1 - 3.5 (6H, m),
7.08 (2H, d, J=10Hz),
7.45 (2H, d, J=10Hz).

Example 3:

4-Cyanoquinuclidine sulfate

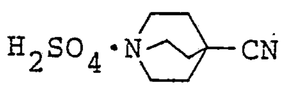

Sodium amide (7.6 g) was suspended in 1.2-dimethoxyethane (90 m ℓ ), followed by a dropwise addition of a mixed solution of acetonitrile (2.6 g), trichloroethylamine (10.0 g) and 1,2-dimethoxyethane (30 m ℓ ) under vigorous stirring over 20 minutes. Chilled water (30 m ℓ ) and chloroform (50 m ℓ ) were added to the reaction mixture and the organic layer was collected. Chloroform was then added to the water layer to extract same. The extract was combined with the organic layer, and anhydrous magnesium sulfate (20 g) was added to the resulting mixture to dry same. The magnesium sulfate was filtered off, and the filtrate was added with a mixture of sulfuric acid (3.4 g) and methanol (10 m ℓ ) and then concentrated under reduced pressure. Ethanol (50 m ℓ ) was added to the residue, and the resulting crystals were collected by filtration and dried to obtain 6.1 g of the desired product (yield: 62.0%).
Melting point: 155 - 160°C.

**Claims**

1. A process for the synthesis of 4-cyanoquinuclidine or a salt thereof, which comprises reacting a compound represented by the following formula (I):

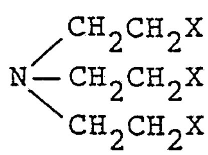

(I)

wherein X means a halogen atom, or a salt thereof with acetonitrile in the presence of a base.

2. The process as claimed in Claim 1, wherein trichloroethylamine and acetonitrile are reacted in the presence of sodium amide.

European Patent Office

# EUROPEAN SEARCH REPORT

Application Number

EP 87 11 7397

## DOCUMENTS CONSIDERED TO BE RELEVANT

| Category | Citation of document with indication, where appropriate, of relevant passages | Relevant to claim | CLASSIFICATION OF THE APPLICATION (Int. Cl.3) |
|---|---|---|---|
| P,A | EP-A-0 213 337 (EISAI CO., LTD.)<br>* columns 9-12, experiments 2, 3 *<br>--- | 1 | C 07 D 453/02 |
| P,A | CHEMICAL ABSTRACTS, vol. 107, no. 21, 23th November 1987, page 743, abstract no. 198112g, Columbus, Ohio, US; & JP - A - 62 16481 (H. YAMAUCHI et al.) 24-01-1987 (Cat. P,A)<br>--- | 1 | |
| A | HELVETICA CHIMICA ACTA, vol. 57, no. 8, 1974, pages 2332-2338; E. CEPPI et al.: "253. Synthese und Basizität 4-substituierter Chinuclidine"<br>* page 2334, last paragraph - page 2335, first paragraph *<br>--- | 1 | |
| D,A | HELVETICA CHIMICA ACTA, vol. 37, no. 6, 1954, pages 1681-1688, C.A. GROB et al.: "194. Untersuchung in der Chinuclidin-Reihe. 1. Mitteilung. Synthese und C-Alkylierng des 1-Methyl-4-cyano-piperidins"<br>--- | 1 | TECHNICAL FIELDS SEARCHED (Int. Cl.3) |
| A | CHEMICAL ABSTRACTS, vol. 53, no. 8, 25th April 1959, column 7204i-7205c, Columbus, Ohio, US; & CH - A - 329 203<br>----- | 1 | C 07 D 453/00 |

The present search report has been drawn up for all claims

| Place of search | Date of completion of the search | Examiner |
|---|---|---|
| BERLIN | 25-02-1988 | HASS C V F |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)